# EUROPÄISCHE PATENTSCHRIFT

(11) **EP 0 581 924 B1**
(45) Veröffentlichungstag und Bekanntmachung des Hinweises auf die Patenterteilung: **24.03.1999**
(21) Anmeldenummer: 93903139.9
(22) Anmeldetag: 12.02.1993
(51) Int. Cl.: A61M 5/315

(54) **INJEKTIONSGERÄT**
INJECTION DEVICE
APPAREIL D'INJECTION

(30) Priorität: 21.02.1992 CH 534/92
(43) Veröffentlichungstag der Anmeldung: 09.02.1994
(73) Patentinhaber: MEDIMPEX ETS., FL-9496 Balzers (LI)
(72) Erfinder: MICHEL, Peter, CH-3400 Burgdorf (CH); KIRCHHOFER, Fritz, CH-3454 Sumiswald (CH)
(74) Vertreter: Lusuardi, Werther Giovanni, Dr.
(86) Internationale Anmeldenummer: CH9300037
(87) Internationale Veröffentlichungsnummer: WO9316740

(56) Entgegenhaltungen:
- WO-A-87/02895
- WO-A-90/09202

## Beschreibung

Die Erfindung betrifft ein Injektionsgerät gemäss dem Oberbegriff des Patentanspruches 1.

Ein Injektionsgerät dieser Art (im folgenden oft kurz "Gerät" genannt) ist aus der WO 87/02895 bekannt.
Das bekannte Injektionsgerät dient zum Injizieren jeweils wählbarer Flüssigkeitsmengen aus einer mit einem Kolben ausgerüsteten Ampulle. Es besitzt eine manuell antreibbare Betätigungseinrichtung, die unter anderem ein in der Vorschubrichtung des Kolbens bewegbares, vornehmlich als Stab mit einem Flansch ausgebildetes Abtriebsglied und einen sowohl axial wie auch in Umdrehungen bewegbaren Bedienknopf aufweist. Ein Antriebselement, an dem das Abtriebsglied bewegbar gelagert ist, ist in der Vorschubrichtung des Kolbens aus einer Ruhelage in eine Endlage und wieder zurück in die Ruhelage verschiebbar. In der Ruhelage des Antriebselements ist das dann sich in einem Abstand vom Kolben befindliche Abtriebsglied in der Vorschubrichtung entsprechend einem für die jeweils zu injizierende Flüssigkeitsmenge erforderlichen Kolbenweg bewegbar, ohne am Kolben anzustossen. Der Kolbenweg wird durch die Grösse der Drehung am Bedienknopf bestimmt. Durch einen Rastermechanismus, der beim Überfahren jeder Rasterung ein kleines akustisches Signal abgibt, kann der Patient die Grösse der neuen Injektionsdosis durch Abzählen bestimmen. Während des Vorschubs des Antriebselementes aus der Ruhelage in die Endlage stösst das Abtriebsglied am Kolben an und verschiebt diesen entsprechend dem vorgewählten Kolbenweg. Das Antriebselement wird durch eine Feder in der Ruhelage gehalten und ist gegen die Kraft dieser Feder in die Endlage verschiebbar.

In der Anwendung befriedigt dieses Injektionsgerät aus den folgenden Gründen noch nicht ganz.

Die Abmessungen eines Injektionsgerätes sind ungefähr auf die eines Füllfederhalters festgelegt, da das Gerät von Patienten, bei denen zu jeder beliebigen Zeit eine Injektion notwendig sein kann, dauernd getragen werden soll. Das Verhältnis zwischen der Länge der Betätigungseinrichtung zur maximalen Injektionsdosis dieses bekannten Gerätes reicht aber noch nicht ganz aus, da die von dem Gerät maximal in einer Injektion abgebbare Injektionsdosis bei gewissen Patienten noch zu klein ist. Die Ampullengrösse und die Konzentrationen der verwendeten Injektionslösungen sind aber seit langem festgelegt, Änderungen daran würden auf sehr grossen Widerstand durch die Arzneimittelhersteller stossen. Es ist daher ein Gerät mit der gegebenen Gesamtlänge erwünscht, mit dem auch grössere Maximaldosen injizierbar sind.

Die Erfindung soll somit die Aufgabe lösen, ein Injektionsgerät zu schaffen, das in den Abmessungen etwa gleich gross ist wie das bekannte Gerät, mit dem aber grössere Injektionsdosen verabreichbar sind.

Diese Aufgabe wird durch die im Kennzeichenteil des Anspruchs 1 aufgeführten Merkmale gelöst, die weiteren Ansprüche betreffen vorteilhafte Ausführungsformen.

Die Erfindung wird an Hand der Zeichnung dargestellt, dabei zeigen die
- Fig. 1: einen Längsschnitt durch ein Injektionsgerät nach dem Stand der Technik,
- Fig. 2: einen Längsschnitt durch ein erfindungsgemässes Injektionsgerät,
- Fig. 3: den Querschnitt A - A des Gerätes nach Fig. 2,
- Fig. 4: den Querschnitt B - B des Gerätes nach Fig. 2,
- Fig. 5: den Querschnitt C - C des Gerätes nach Fig. 2 und
- Fig. 6: eine Abwicklung längs der Richtung D - D in der Fig. 2.

In der Zeichnung sind gleichartige Teile mit denselben Beizeichen bezeichnet.

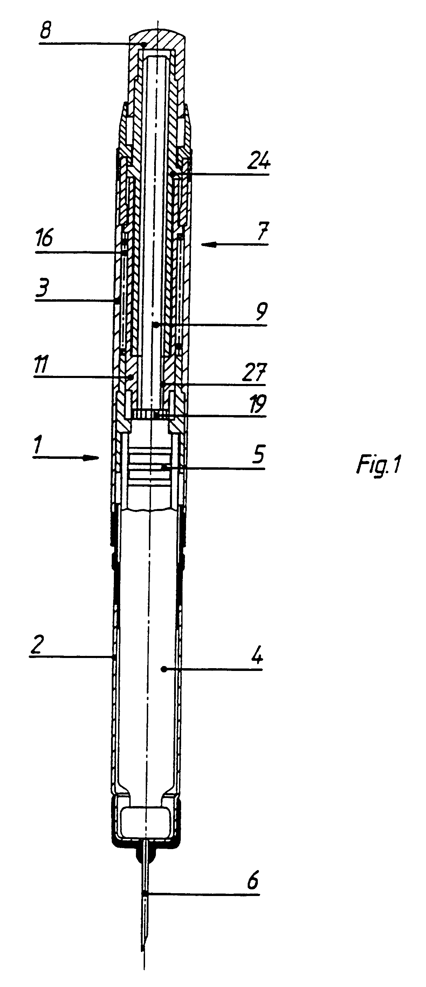

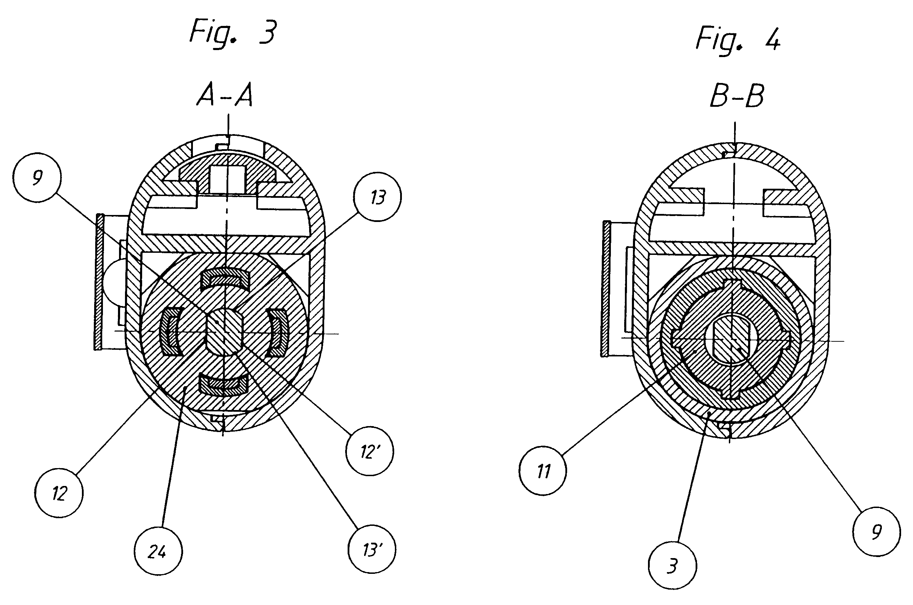

Ein bekanntes Injektionsgerät 1 nach der Fig. 1 (und den Figuren 1 bis 7 der WO 87/02895) wird im folgenden im Detail beschrieben. Es enthält in einem vorderen Teil 2 eine austauschbare Ampulle 4 mit einem Kolben 5, mit dem die zu injizierende Substanz durch eine Injektionsnadel 6 austreibbar ist. In seinem hinteren Teil 3 besitzt es eine von Hand bedienbare, röhrenförmig aufgebaute Betätigungseinrichtung 7. Diese weist einen Bedienknopf 8, eine vornehmlich als ein Stab 9 mit einem Flansch 19 ausgebildete Abtriebsvorrichtung, ein Führungselement 24 und ein Antriebselement 11 auf.

Der Stab 9 besitzt an beiden Seiten plane Flächen, weist im Übrigen einen kreisförmigen Querschnitt auf und trägt auf den kreisförmigen Flächen ein Gewinde. Mit diesem Gewinde sitzt er in einem Muttergewinde 27 des Antriebselementes 11, letzteres ist drehfest im Gerät 1 angebracht. Das Antriebselement 11 ist zusammen mit der gesamten Betätigungseinrichtung 7 durch axiales Betätigen des Bedienknopfes 8 und Vermittlung dieser Bewegung über das Führungselement 24 gegen die Kraft einer Feder 16 von einer Ruheposition in eine Endposition bringbar. Auch der Stab 9 macht diese Bewegung mit. Ein vorne am Stab 9 befindlicher Flansch 19 drückt dabei einen Kolben 5 der Ampulle 4 nach vorne und bewirkt die Injektion.

Der Stab 9 sitzt ferner drehfest, aber axial beweglich im Führungselement 24, da dieses mindestens über einen Abschnitt (bis auf Gleittoleranzen) innen denselben Querschnitt aufweist, den auch der Stab 9 besitzt. Das Führungselement 24 ist seinerseits drehfest mit dem Bedienkopf 8 verbunden. Der Bedienkopf 8, das Führungselement 24 und der Stab 9 lassen sich - nur in der Ruhestellung der Betätigungseinrichtung 7 - drehen. Der Stab 9 dreht sich dabei im Muttergewinde 27 des im Gerät 1 drehfest angeordneten Antriebselementes 11 und verstellt sich nach vorne oder - bei Umkehr der Drehrichtung des Bedienknopfes 8 - nach hinten.

Wurde zuvor eine Injektion durchgeführt, lässt sich die Menge der bei der nächsten Injektion verwendeten Substanz festlegen, indem der Stab 9 durch Drehen am Bedienknopf 8 um eine bestimmte Länge nach vorne gestellt wird. Diese Verstellung ist durch Anschläge in der Betätigungseinrichtung 7 so begrenzt, dass der Flansch 19 beim Einstellen der Injektionsmenge den Kolben 5 der Ampulle 4 nicht berühren kann.

Um die der zu injizierenden Menge entsprechende Verstellänge zu bestimmen, gibt das beschriebene Gerät 1 bei jeder Vierteldrehung des Bedienknopfes 8 ein deutliches, von einem zwischen der die Drehbewegung ausfuhrenden Führungselement 24 und dem in dem im Gerät 1 drehfest angeordneten Antriebselement 11 befindlichen Drehraster herrührendes Geräusch von sich, so dass für den Patienten die zu injizierende Menge durch Zählen der Rastergeräusche einstellbar ist.

Bei dem bekannten Injektionsgerät 1 befindet sich das drehfest im Gerät 1 eingebaute Antriebselement 11 vor dem Führungselement 24. Im Antriebselement 11 sitzt im Muttergewinde 27, nur durch Drehung verschiebbar gelagert, der Stab 9, der beim Übergang von der Ruhestellung zur Endstellung vorgeschoben werden muss. Daher muss bei dem bekannten Gerät auch das Antriebselement 11 beim Übergang von der Ruhestellung zur Endstellung vorgeschoben werden.

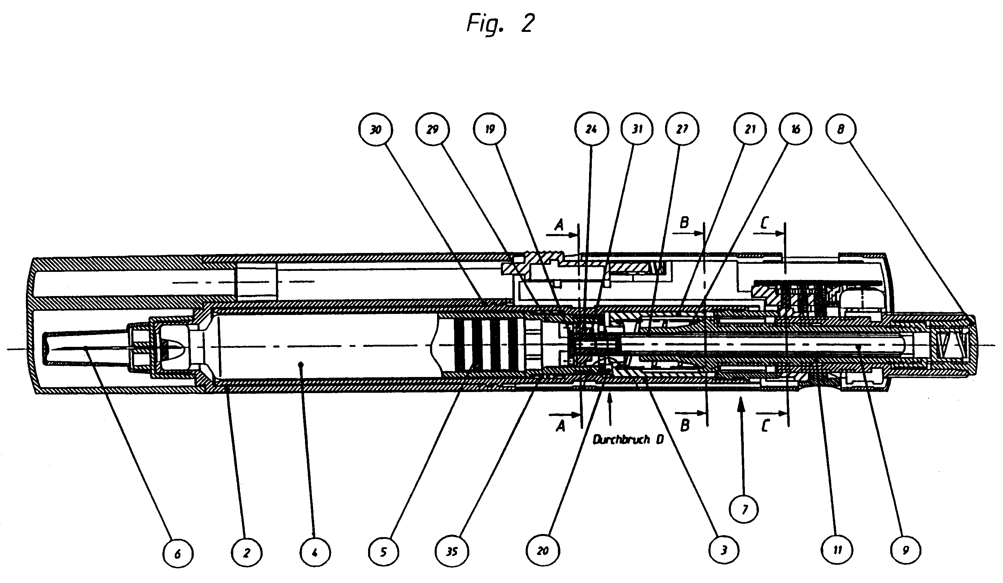

Das erfindungsgemässe Injektionsgerät zeigen die Figuren 2 bis 6. Auch es besitzt einen vorderen Teil 2, in das eine Ampulle 4 mit einem Kolben 5 und mit einer Injektionsnadel 6 einschiebbar ist. Der vordere Teil 2 und ein hinterer Teil 3 sind durch ein Grobgewinde 30 verbunden, so dass man die Ampulle 4 leicht ersetzen kann. Im hinteren Teil 3 befindet sich eine Betätigungseinrichtung 7, die im wesentlichen aus einem Bedienknopf 8, einem Antriebselement 11, einem Führungselement 24 und einem Abtriebsglied 9,19, welches aus einem Stab 9 und einem Flansch 19 zusammengesetzt ist, besteht. Der Stab 9 besitzt an seinen Längsseiten zwei plane Flächen 12,12' und zwei Kreisflächen 13,13', in die Gewindeteile eingeschnitten sind. Statt der Ampulle 4 kann auch ein anderes Gefäss mit Kolben 5 verwendet werden

Das Antriebselement 11 ist röhrenförmig und mit dem Bedienknopf 8 fest gegen Verdrehung verbunden. In seinem Inneren liegt der Stab 9. Am vorderen Ende trägt es ein Muttergewinde 27, das in die Gewindeteile des Stabes 9 eingreift. Der Stab 9 geht durch das Antriebselement 11 und das Führungselement 24 hindurch. Das Führungselement 24 ist fest mit dem hinteren Teil 3 des Gerätes verbunden und kann daher weder eine axiale noch eine Rotationsbewegung ausführen. Die Öffnung im Führungselement 24, durch die der Stab 9 hindurchgeht, besitzt denselben, um die notwendigen Toleranzen vergrösserten Querschnitt wie der Stab 9, also zwei plane und zwei kreisförmige Umfangstelle, so dass der Stab 9 durch die Öffnung des Führungselementes 24 nur axiale, keine Rotationsbewegungen ausführen kann.

Der Bedienknopf 8 wird manuell bedient und kann axiale und, in einer Ruhestellung der Betätigungseinrichtung 7, auch Drehbewegungen ausführen. Wird er durch Druck axial betätigt, so verschiebt er das Antriebselement 11 bis zu einer Endstellung, die durch einen Anschlag 31, beispielsweise des Antriebselementes 11 am Führungselement 24, das gegen axiale Verschiebungen fest mit dem hinteren Teil 3 des Gerätes verbunden ist.

Im Antriebselement 11 sitzt im Muttergewinde 27 der Stab 9, auf den daher diese axiale Bewegung übertragen wird. Diese axiale Bewegung wird auch durch das dreh- und axial fest mit dem hinteren Teil 3 verbundene Führungselement 24 nicht behindert, da die Öffnung des Führungselementes 24 und der Stab 9 denselben Querschnitt besitzen und sich drehfest ineinander axial bewegen, jedoch keine Rotationsbewegung ausführen können.

Diese axiale Bewegung wird gegen die Kraft einer Feder 16 ausgeführt, die in einer Aussparung zwischen dem die axiale Bewegung ausführenden Antriebselement 11 und einem Hülsenteil 21 eines Drehrasters 20 liegt. Die erste Feder 16 bringt die Betätigungseinrichtung 7 wieder in die Ruhestellung zurück.

Wird der Bedienknopf 8 zur Einstellung der nächsten Injektionsdosis gedreht, so dreht sich das Antriebselement 11 mit. Diese Drehbewegung kann aber nicht auf den Stab 9 übertragen werden, da dieser im Führungselement 24 drehfest gelagert ist. Durch das sich drehende Muttergewinde 27 des Führungselements 11 wird über die Gewindeteile an den Kreisflächen 13,13' des Stabes 9 dieser drehfest nach vorne (oder bei Umkehr der Drehrichtung am Bedienknopf nach hinten) getrieben und so der Flansch 19 in die Stellung gebracht, die die nächste abzugebende Injektionsdosis erfordert, d.h. der Abstand des Flansches 19 vom Kolben 5 wird entsprechend verringert.

Nun wird durch Druck auf den Bedienknopf 8 die Betätigungseinrichtung 7 aus der Ruhestellung in die Endlage vorgeschoben. Der Flansch 19 stösst dabei am Kolben 5 an und nimmt ihn auf dem eingestellten Kolbenweg mit, wodurch das im voraus eingestellte Volumen der Injektionsflüssigkeit durch die Injektionsnadel 6 ausgestossen wird. Der Weg des Flansches 19 von der Ruhelage in die Endlage der Betätigungseinrichtung 7 bleibt dabei immer gleich und entspricht der konstanten Distanz, um die der Flansch 19 vor Einstellung der Injektionsdosis vom Kolben 5 getrennt ist.

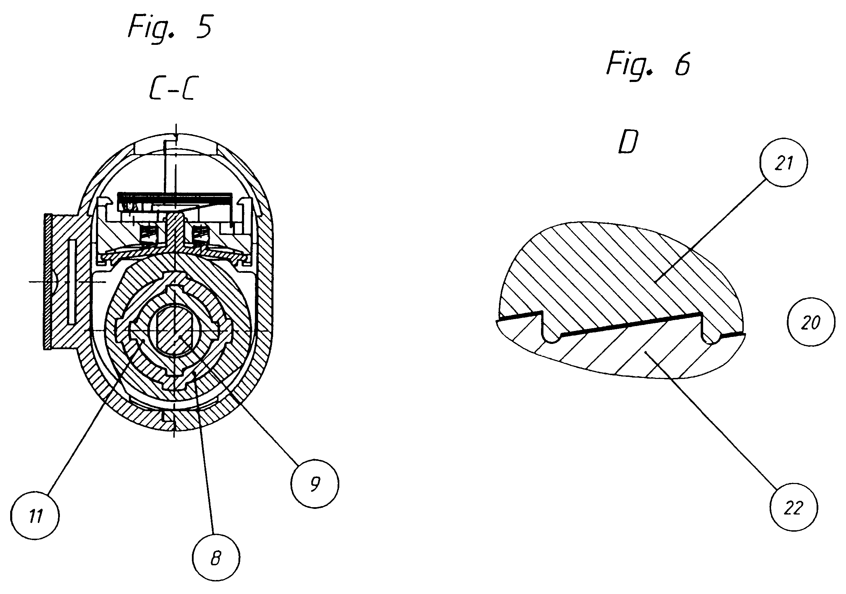

Zwischen den drehfesten und den drehbaren Teilen des Injektionsgerätes befindet sich ein Drehraster 20. Es ist in der Fig. 6 im Durchbruch D als Abwicklung auf neutralem Durchmesser dargestellt. Bei ihm wirkt der Ampullenhalter 29 mit durch das Führungselement 24 gehenden Vorsprüngen 22 mit dem Hülsenteil 21 zusammen. Beide sind an ihren aneinanderliegenden Enden beispielsweise sägezahnartig (siehe Fig. 6) ausgebildet und bilden so das Drehraster 20. Das Hülsenteil 21 wird durch die an dem Antriebselement 11 anliegende erste Feder 16 gegen die Vorsprünge des Ampullenhalters 29 gedrückt, so dass sich das Drehraster 20 in der einen Drehrichtung leicht, in der anderen Drehrichtung nicht bewegt.

Die Drehrichtung, mit der der Bedienknopf 8 das Abtriebsglied 9, 19 in die der nächsten Injektionsdosis entsprechende Stellung bringt, ist die Drehrichtung mit kleinem Widerstand. Soll beim Auswechseln einer Ampulle 4 der Stab 9 zurückgedreht werden, so wird der Ampullenhalter 29 beim Herausnehmen der alten Ampulle 4 durch eine Feder 35, die zwischen dem hinteren Teil 3 und dem Ampullenhalter 29 sitzt, nach vorne geschoben und damit das Drehraster 20 in beiden Drehrichtungen ganz frei gegeben, so dass der Stab 9 leicht mit Hilfe des Bedienknopfes 8 in die Ausgangsstellung zurückgedreht werden kann.

Bei dem erfindungsgemässen Injektionsgerät liegt das ortsfeste Führungselement 24 vor dem Antriebselement 11. Nur das Antriebselement wird beim Übergang von der Ruhestellung in die Endstellung und zurück axial verschoben, das vorne liegende Führungselement 24 bleibt von dieser Verschiebung unberührt. Dadurch wird die gesamte Betätigungseinrichtung 7 kürzer als die des vorbekannten Gerätes, es ist bei gleicher Gesamtbaulänge beider Injektionsgeräte in dem erfindungsgemässen Gerät eine grössere Verschiebelänge zwischen der Ruhelage und der Endlage für den mit dem Stab 9 verbundenen Flansch 19 einrichtbar. Das erfindungsgemässe Injektionsgerät erfüllt somit die gestellte Aufgabe, eine grössere Injektionsdosis einstellbar zu machen als bei dem vorbekannten Gerät.

## Patentansprüche

1. Injektionsgerät zum Injizieren jeweils wählbarer Flüssigkeitsmengen aus einem mit einem Kolben (5) ausgerüsteten Flüssigkeitsbehälter (4), insbesondere einer Ampulle (4), mit einer manuell antreibbaren röhrenartigen Betätigungseinrichtung (7),
- die ein in der Vorschubrichtung des Kolbens (5) bewegbares Abtriebsglied (9, 19), einen Bedienknopf (8), an dem axiale und rotierende Bewegungen ausführbar sind, ferner ein Antriebselement (11), das drehfähig gegen das in ihm gelagerte Abtriebsglied (9, 19) ist, und ein Führungselement (24) für das Abtriebsglied (9, 19) besitzt,
- bei der bei axialer Bewegung des Bedienknopfes (8) das Antriebselement (11) in der Vorschubrichtung des Kolbens (5) aus einer Ruhelage in eine Endlage und wieder zurück verschiebbar ist, und diese Bewegung auf das mit dem Antriebselement (11) verbundene Abtriebsglied (9, 19) übertragbar ist,
- bei der in der Ruhelage durch eine Drehbewegung des Bedienknopfes (8) das vom Kolben (5) distanzierte Abtriebsglied (9, 19) durch das Antriebselement (11) in der Vorschubrichtung entsprechend einem für die jeweils zu injizierende Flüssigkeitsmenge erforderlichen Kolbenweg bewegbar ist, wobei das Abtriebsglied (9, 19) vom Kolben (5) distanziert bleibt und
- wobei während des Überganges des Antriebselementes (11) von der Ruhelage zur Endlage das Abtriebsglied (9, 19) am Kolben (5) anliegt, wobei der Kolben (5) um einen vorgewählten Kolbenweg verschiebbar ist,
**dadurch gekennzeichnet, dass**
- das Antriebselement (11) drehfest mit dem Bedienknopf (8) verbunden ist und für beide alle Bewegungen gemeinsam ausführbar sind,
- das Führungselement (24) fest mit dem hinteren Teil (3) des Gerätes (1) verbunden ist,
- das Abtriebsglied (9, 19) im Führungselement (24) drehfest gelagert ist,
- in der Betätigungseinrichtung (7) das Führungselement (24), in Vorschubrichtung gesehen, vor dem Antriebselement (11) angeordnet ist.

2. Gerät nach Anspruch 1, dadurch gekennzeichnet, dass das Antriebselement (11) durch eine erste Feder (16) in der Ruhelage gehalten und gegen die Kraft der ersten Feder (16) in die Endlage verschiebbar ist.

3. Gerät nach Anspruch 1, dadurch gekennzeichnet, dass das Abtriebsglied (9, 19) einen Stab (9) besitzt, der vorne einen Flansch trägt, auf zwei gegenüber liegenden Seiten plane Flächen (12,12'), im übrigen Kreisflächen (13,13') mit Gewinden aufweist.

4. Gerät nach Anspruch 1, dadurch gekennzeichnet, dass sich zwischen dem Führungselement (24) oder mit diesem drehfest verbundenen Teilen und dem Antriebselement (11) oder mit diesem drehfest verbundenen Teilen ein Drehraster (20) befindet.

5. Gerät nach Anspruch 4, dadurch gekennzeichnet, dass das Drehraster (20) ein Sägezahnraster ist.

6. Gerät nach Anspruch 4, dadurch gekennzeichnet, dass die Teile (21,22) des Drehrasters (20) sich gegen Federn (16, 35), die entweder zwischen drehfesten Teilen wie dem Ampullenhalter (29) und dem hinteren Teil (3) oder zwischen drehbaren Teilen, wie dem Antriebselement (11) und dem Hülsenteil 21 befinden, gegeneinander bewegbar sind.

7. Gerät nach Anspruch 6, dadurch gekennzeichnet, dass die Drehrichtung des Bedienknopfes (8), in der die Injektionsdosis eingestellt wird, die Richtung ist, in der das Drehraster (20) mit kleinem Widerstand läuft.

8. Gerät nach Anspruch 4, dadurch gekennzeichnet, dass das Drehraster (20) entlastbar ist, indem durch Entnahme der Ampulle (4) aus dem Gerät der sonst gegen die Ampulle (4) mittels der Feder (35) drückende Ampullenhalter (34) nach vorne geschoben wird, so dass der Bedienknopf (8) und mit ihm das Antriebselement (11) ohne Widerstand in beiden Richtungen drehbar sind und auf diese Weise das Abtriebsglied (9, 19) in seine Ausgangstellung zurückdrehbar ist.

## Claims

1. Injection device to inject particular, selectable quantities of liquid from a liquid receptacle (4), in particular an ampoule (4), fitted with a plunger (5) and comprising a manual, tubular actuation system (7),
-- comprising an output member (9, 19) displaceable in the direction of advance of the plunger (5), an operating head (8) which can be moved axially and rotationally, further a drive element (11) which can rotate relative to the output member (9, 19) seated inside it, and a guide element (24) for the output member (9, 19)
-- wherein upon axial displacement of the operating head (8) the drive element (11) is displaceable in the direction of advance of the plunger (5) from a rest position into an end position and back and where this motion is transmitted to the output member (9, 19) connected to the drive element (11),
-- wherein for the rest position the output member (9, 19) moved away from the plunger (5) by rotating the operating head (8) can be displaced by the drive element (11) in the direction of advance in relation to a plunger stroke required for the related particular quantity of liquid which must be injected without the output member (9, 19) thereby touching the plunger, and
-- wherein during the transit of the drive element (11) from the rest position to the end position the output member (9, 19) impacts the plunger (5) which is thereby displaceable along a pre-set plunger path,
**characterized in that**
-- the drive element (11) is non-rotatably connected to the operating head (8) and both are apt to carry out jointly all their motions,
-- the guide element (24) is rigidly affixed to the rear part (3) of the device (1), -- the output member (9, 19) is non-rotatably supported in the guide element (24),
-- the guide element (24) is in front of the drive element (11) in the direction of advance and inside the actuation system (7).

2. Device defined in claim 1, characterized in that the drive element (11) is kept in the rest position by a first spring (16) and is displaceable into the end position against the force of this first spring (16).

3. Device defined in claim 1, characterized in that the output member (9, 19) comprises a rod (9) fitted with a front flange and has planar surfaces (12, 12') at two mutually opposite sides and elsewhere threaded circular areas (13, 13').

4. Device defined in claim 1, characterized in that a rotary detent (20) is present between the guide element (24) or parts non-rotatably affixed to it and the drive element (11) or parts non-rotatably affixed to it.

5. Device defined in claim 4, characterized in that the rotary detent (20) is a serrated detent.

6. Device defined in claim 4, characterized in that the parts (21, 22) of the rotary detent (20) are mutually displaceable against springs (16, 35) located either between the non-rotatable parts such as the ampoule holder (29) and the rear part (3) or between rotary parts such as the drive element (11) and the sleeve part (21).

7. Device defined in claim 6, characterized in that the rotational direction of the operating head (8) setting the injection dose is that direction allowing the rotary detent (20) to move with little drag.

8. Device defined in claim 4, characterized in that the rotary detent (20) can be relieved by removing the ampoule (4) from the device (1) in that the ampoule holder (34) -- which otherwise presses by means of the spring (35) against the ampoule (4) -- is being advanced, whereby the operating head (8) and with it the drive element (11) are rotatable without drag in both directions and the output member (9, 19) thereby is rotatable back into its initial position.

## Revendications

1. Appareil d'injection pour injecter des quantités chaque fois sélectionnables d'un liquide pris dans un récipient (4) à liquide équipé d'un piston (5), en particulier une ampoule (4), et comportant un dispositif d'actionnement (7) de type tubulaire apte à être entraîné manuellement,
- lequel dispositif d'actionnement possède un élément entraîné (9, 19) qui peut être déplacé dans le sens d'avancement du piston (5), une tête de commande (8) qui peut exécuter des déplacements axiaux et en rotation, et en outre un élément d'entraînement (11) qui peut tourner par rapport à l'élément entraîné (9, 19) monté en lui, et un élément de guidage (24) de l'élément entraîné (9, 19),
- dans lequel, lors d'un déplacement axial de la tête de commande (8), l'élément d'entraînement (11) peut être déplacé dans le sens d'avancement du piston (5) d'une position de repos à une position finale et inversement, et ce déplacement peut être transmis à l'élément entraîné (9, 19) relié à l'élément d'entraînement (11),
- dans lequel l'élément entraîné (9, 19) qui, dans la position de repos, est écarté du piston (5) par un déplacement de rotation de la tête de commande (8), peut être déplacé dans le sens d'avancement par l'élément d'entraînement (11), en correspondance avec la course du piston nécessaire pour la quantité de liquide à injecter, l'élément entraîné (9, 19) restant à distance du piston (5), et
- dans lequel, pendant le passage de l'élément d'entraînement (11) de la position de repos à la position finale, l'élément entraîné (9, 19) repose contre le piston (5), et le piston (5) peut être déplacé sur une course de piston présélectionnée,
caractérisé en ce que:
- l'élément d'entraînement (11) est relié à rotation solidaire à la tête de commande (8) et tous les déplacements peuvent être exécutés en commun par l'élément d'entraînement et la tête de commande,
- l'élément de guidage (24) est solidaire de la partie arrière (3) de l'appareil (1),
- l'élément entraîné (9, 19) est monté dans l'élément de guidage (24) de manière à ne pas pouvoir tourner,
- vu dans le sens d'avancement, l'élément de guidage (24) est disposé dans le dispositif d'actionnement (7) en avant de l'élément d'entraînement (11).

2. Appareil selon la revendication 1, caractérisé en ce que l'élément d'entraînement (11) est maintenu dans la position de repos par un premier ressort (16) et peut être déplacé dans la position finale en opposition à la force du premier ressort (16).

3. Appareil selon la revendication 1, caractérisé en ce que l'élément entraîné (9, 19) possède un barreau (9) qui porte une bride à l'avant, qui présente des surfaces planes (12, 12') sur deux côtés opposés, et pour le reste des surfaces circulaires (13, 13') dotées de filets.

4. Appareil selon la revendication 1, caractérisé en ce qu'un cliquet de rotation (20) est situé entre l'élément de guidage (24) ou des pièces reliées à rotation solidaire à ce dernier et l'élément d'entraînement (11) ou des pièces reliées à rotation solidaire à ce dernier.

5. Appareil selon la revendication 4, caractérisé en ce que le cliquet de rotation (20) est un cliquet en dents de scie.

6. Appareil selon la revendication 4, caractérisé en ce que les parties (21, 22) du cliquet de rotation (20) peuvent se déplacer l'une par rapport à l'autre en opposition à des ressorts (16, 35) qui sont situés soit entre des parties empêchées de tourner, telles que le support d'ampoule (29) et la partie arrière (3), ou entre des parties tournantes, comme l'élément d'entraînement (11) et la pièce formant manchon (21).

7. Appareil selon la revendication 6, caractérisé en ce que le sens de la rotation de la tête de commande (8) par laquelle la dose d'injection est réglée est le sens dans lequel les cliquets de rotation (20) se déplacent avec une faible résistance.

8. Appareil selon la revendication 4, caractérisé en se que l'enlèvement de l'ampoule (4) hors de l'appareil permet de décharger le cliquet de rotation (20) en déplaçant vers l'avant le support d'ampoule (34) qui, sinon, repousse l'ampoule (4) au moyen du ressort (35), de telle sorte que la tête de commande (8), et avec elle l'élément d'entraînement (11), peuvent être tournés sans résistance dans les deux sens, l'élément entraîné (9, 19) pouvant de cette manière être ramené dans sa position de départ.
